(19) Europäisches Patentamt
European Patent Office
Office européen des brevets

(11) **EP 4 449 878 A1**

(12) **EUROPEAN PATENT APPLICATION**

(43) Date of publication:
**23.10.2024 Bulletin 2024/43**

(21) Application number: **23168563.7**

(22) Date of filing: **18.04.2023**

(51) International Patent Classification (IPC):
**A23L 2/44** (2006.01)    **A23L 3/3508** (2006.01)
**C12P 7/52** (2006.01)

(52) Cooperative Patent Classification (CPC):
**A23L 3/3508; C12P 7/52;** A23L 2/44;
C12R 2001/01

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Purac Biochem B.V.**
**4206 AC Gorinchem (NL)**

(72) Inventor: **The designation of the inventor has not yet been filed**

(74) Representative: **Nederlandsch Octrooibureau**
**P.O. Box 29720**
**2502 LS The Hague (NL)**

(54) **LIQUID PROPIONATE-CONTAINING FERMENTATE AND PROCESS FOR THE MANUFACTURE THEREOF**

(57)    The invention relates to a liquid fermentate having a dry matter content of at least 15 g/L and comprising:
• a mmol of propionate per gram of dry matter;
• b mmol of acetate per gram of dry matter;
• c. mmol of succinate per gram of dry matter;
• x mmol of $Na^+$ per gram of dry matter;
• y mmol of $K^+$ per gram of dry matter;
• z mmol of $Ca^{2+}$ per gram of dry matter;
said fermentate comprising DNA originating from a propionic acid producing micro-organism; wherein:

• $$6 \leq a \leq 9;$$

• $$1 \leq b \leq 5;$$

• $$0.1 \leq c \leq 1.0;$$

• $$8 \leq x \leq 18;$$

• $$8 \leq y \leq 18;$$

• $$4 \leq z \leq 9;$$

• $$c/a \leq 0.1;$$

• $$0.5 \leq (a+b+2c)/(x+y+2z) \leq 1.2.$$

The invention also provides a process of producing a liquid propionate-containing fermentate, said process using a combination of (i) monosaccharide selected from glucose, fructose and combinations thereof and (ii) lactate as carbohydrate substrate

EP 4 449 878 A1

**Description**

## TECHNICAL FIELD OF THE INVENTION

The present invention relates to a process of producing a liquid propionate-containing fermentate, said process comprising:

- providing a fermentation medium comprising fermentable carbohydrates;
- inoculating the fermentation medium with a propionic acid producing micro-organism;
- incubating the inoculated aqueous liquid for at least 20 hours to produce a fermentate; and
- removing biomass from the fermentate.

The invention further relates to a liquid fermentate having a dry matter content of at least 15 g/L, said fermentate containing propionate, acetate, succinate, alkalimetal cation and DNA originating from a propionic acid producing micro-organism.

Also provided is a propionate concentrate having a dry matter content of 40-65 wt.% and containing propionate, acetate, succinate, lactate, alkalimetal cation and DNA, said concentrate having a pH in the range of 4.0-6.0 when diluted 1:9 (v/v) with demi-water, and comprising DNA originating from a propionic acid producing micro-organism. The propionate concentrate of the present invention may suitably be applied in foodstuffs, nutritional products and beverages as an antimicrobial agent. The propionate concentrate is particularly suitable for use as a preservative in baked products and processed meat.

The invention also provides a method of preparing a product selected from a food product, a nutritional product and a beverage, said method comprising application of the propionate concentrate of the present invention.

## BACKGROUND OF THE INVENTION

Preservatives extend the shelf life of baked goods by inhibiting the growth of rope bacteria and moulds. Rope bacteria (*Bacillus mesentericus*) can be carried through the baking process and cause an unpleasant texture and odour inside yeast-raised products like bread. Moulds can contaminate products after baking and then grow on the outside surface. Mould contamination is considered a serious problem amongst bakers, and conditions commonly found in baking present near-optimal conditions for mould growth.

Acidity decreases the chance of spoilage. Most preservatives have acids as an active ingredient. Preservatives can be classified according to the kind of acid they contain: mineral acid, organic acid, or fatty acid. Mineral acids (like sulfuric, hydrochloric, and phosphoric) are the strongest acids and so have the greatest effect on pH. But they work only by lowering pH and do not contain any other inhibitors. Organic acids (like lactic, benzoic, and tartaric) have less effect on pH than do mineral acids but are better preservatives because they have an additional inhibitory activity. Fatty acids (like acetic, sorbic, and propionic) are a specific kind of organic acid with an even stronger inhibitory activity.

Calcium propionate is used as a preservative in a wide variety of products, including: bread, other baked goods, processed meat, whey, and other dairy products. Propionates prevent microbes from producing the energy they need, like benzoates do. However, unlike benzoates, propionates do not require an acidic environment.

Propionate is a commercially valuable carboxylic acid produced through microbial fermentation. Biological propionate production is limited by high downstream purification costs.

WO 2010/097362 describes a method for manufacturing a mixed solution comprising propionate and acetate, which comprises the steps of

- providing a fermentation product comprising propionate, acetate, and carbonate-related compounds,
- adding acid to the fermentation product to obtain an acidified fermentation product comprising propionate and acetate with a pH in the range of 2.5 to below 8.

WO 2016/146721 describes a process for manufacturing propionate products through fermentation, comprising the steps of:

- fermenting a carbon source selected from sugars and lactate in a fermentation medium by means of a propionic acid producing microorganism to provide a first fermentation broth comprising a propionate salt,
- recovering propionic acid producing microorganism from the first fermentation broth,
- subjecting the first fermentation broth from which propionic acid producing microorganism have been recovered to a water removal step to form a first propionate salt product,
- fermenting a carbon source comprising glycerol with the propionic acid producing microorganism recovered from the first fermentation broth in the presence of an inorganic alkaline salt to provide a second fermentation broth comprising a propionate salt,

- subjecting the second fermentation broth to a purification step comprising at least one precipitation step, to form a second propionate salt product.

WO 2019/108064 describes an organic acid preservative system comprising an acetate component and a propionate component, wherein the acetate component and the propionate component make up at least 85 wt.% of the total amount of carboxylic acids and carboxylic acid salts in the preservative system and wherein the propionate component makes up 12 to 50 wt.% of said total amount of carboxylic acids and carboxylic acid salts.

## SUMMARY OF THE INVENTION

The inventors have developed a process of producing a liquid propionate-containing fermentate that combines low raw material costs with high yield and minimum downstream processing. An essential element of the present invention is the use of two different carbohydrate substrates, i.e. (i) a monosaccharide selected from glucose, fructose and combinations thereof and (ii) lactate.

Accordingly, the process of producing a liquid propionate-containing fermentate according to the present invention comprises the followings step:

a) providing a fermentation medium comprising fermentable carbohydrates selected from monosaccharide, lactate and combinations thereof, said monosaccharide being selected from glucose, fructose and combinations thereof, said lactate being selected from sodium lactate, potassium lactate, calcium lactate and combinations thereof;
b) inoculating the fermentation medium with a propionic acid producing micro-organism;
c) incubating the inoculated aqueous liquid for at least 20 hours to produce a fermentate while adding an alkalising agent to the fermentation medium to maintain pH within the range of 5.0 to 7.5; and
d) removing biomass from the fermentate to produce a clarified liquid fermentate having a dry matter content of at least 1 wt.%;

wherein the total amount of monosaccharide provided in the fermentation medium in the course of steps a), b) and c) exceeds 30 mmol/L and the total amount of lactate provided in the fermentation medium in the course of steps a), b) and c) exceeds 50 mmol/L, said total
amounts being calculated on the basis of the volume of the fermentation medium upon inoculation in step b);
wherein the molar ratio between the total amount of monosaccharide provided in the fermentation medium and the total amount of lactate provided in the fermentation medium is in the range of 1:2.0 to 5.6:1;
wherein the total amount of sodium cation provided in the fermentation medium in the course of steps a), b) and c) equals X mmol/L, the total amount of potassium cation provided in the fermentation medium in the course of steps a), b) and c) equals Y mmol/L and the total amount of calcium cation provided in the fermentation medium in the course of steps a), b) and c) equals Z mmol/L, said total amounts being calculated on the basis of the volume of the clarified liquid fermentate; and
wherein $X + Y + 2Z \geq 100$ mmol/L.

The inventors have discovered that by using a combination of monosaccharide (glucose and/or fructose) and lactate as carbohydrate substrate, not only a high propionate yield can be achieved, but also unwanted sedimentation during downstream processing, notably during concentration, can be minimised. Consequently, after separation of the biomass and subsequent concentration no additional solid-liquid separation step is required to produce a non-turbid fermentation concentrate. Although the inventors do not wish to be bound by theory, it is believed that by using lactate as co-substrate in the fermentative production of propionate, formation of succinate can be suppressed effectively and subsequent sedimentation of succinate is avoided or at least minimised.

Another aspect of the invention relates to a liquid fermentate having a dry matter content of at least 15 g/L and comprising:

- a mmol of propionate per gram of dry matter;
- b mmol of acetate per gram of dry matter;
- c. mmol of succinate per gram of dry matter;
- x mmol of $Na^+$ per gram of dry matter;
- y mmol of $K^+$ per gram of dry matter;
- z mmol of $Ca^{2+}$ per gram of dry matter;

said fermentate comprising DNA originating from a propionic acid producing micro-organism; wherein:

- $6 \le a \le 9$;

- $1 \le b \le 5$;

- $0.1 \le c \le 1.0$;

- $8 \le x \le 18$;

- $8 \le y \le 18$;

- $4 \le z \le 9$;

- $c/a \le 0.1$;

- $0.5 \le (a+b+2c)/(x+y+2z) \le 1.2$.

A further aspect of the invention relates to a propionate concentrate having a dry matter content of 40-65 wt.% and comprising:

- 1.5-3.5 mol/L of propionate;
- 0.4-2 mol/L of acetate;
- 80-240 mmol/L of succinate;
- 0.55-2.2 mol/L of lactate;
- $x'$ mol/L of $Na^+$;
- $y'$ mol/L of $K^+$;
- $z'$ mol/L of $Ca^{2+}$;

said concentrate having a pH in the range of 4.0-6.0 when diluted 1:9 (v/v) with demi-water, and comprising DNA originating from a propionic acid producing micro-organism;
wherein succinate and propionate are present in the concentrate in a molar ratio of less than 1:10;
wherein $2.7 \le x'+y'+2z' \le 6$.

Yet another aspect of the invention concerns a method of preparing a product selected from a food product, a nutritional product and a beverage, said method comprising application of the aforementioned propionate concentrate in a concentration providing 5-100 mmol propionate per kg of the final product.

## DETAILED DESCRIPTION OF THE INVENTION

As explained above, a first aspect of the invention relates to a process of producing a liquid propionate-containing fermentate, said process comprising the followings step:

a) providing a fermentation medium comprising fermentable carbohydrates selected from monosaccharide, lactate and combinations thereof, said monosaccharide being selected from glucose, fructose and combinations thereof, said lactate being selected from sodium lactate, potassium lactate, calcium lactate and combinations thereof;
b) inoculating the fermentation medium with a propionic acid producing micro-organism;
c) incubating the inoculated aqueous liquid for at least 20 hours to produce a fermentate while adding an alkalising agent to the fermentation medium to maintain pH within the range of 5.0 to 7.5; and
d) removing biomass from the fermentate to produce a clarified liquid fermentate having a dry matter content of at least 1 wt.%;

wherein the total amount of monosaccharide provided in the fermentation medium in the course of steps a), b) and c) exceeds 30 mmol/L and the total amount of lactate provided in the fermentation medium in the course of steps a), b) and c) exceeds 50 mmol/L, said total amounts being calculated on the basis of the volume of the fermentation medium upon inoculation in step b);

wherein the molar ratio between the total amount of monosaccharide provided in the fermentation medium and the total amount of lactate provided in the fermentation medium is in the range of 1:2.0 to 5.6:1;

wherein the total amount of sodium cation provided in the fermentation medium in the course of steps a), b) and c) equals X mmol/L, the total amount of potassium cation provided in the fermentation medium in the course of steps a), b) and c) equals Y mmol/L and the total amount of calcium cation provided in the fermentation medium in the course of steps a), b) and c) equals Z mmol/L, said total amounts being calculated on the basis of the volume of the clarified liquid fermentate; and

wherein $X + Y + 2Z \geq 100$ mmol/L, preferably wherein 200 mmol/L $\leq X + Y + 2Z \leq 500$ mmol/L, more preferably wherein 300 mmol/L $\leq X + Y + 2Z \leq 480$ mmol/L.

Unless indicated otherwise, the term "monosaccharide" as used herein refers to glucose, fructose and combinations of glucose and fructose.

The term "propionate" as used herein encompasses propionic acid as well as salts of propionic acid. Likewise, the terms "acetate" and "succinate" also encompass both the acid forms and salt forms.

The total amount of fermentable carbohydrate provided in the fermentation medium as referred to herein means the total amount of fermentable carbohydrate that is provided as substrate to the propionic acid producing micro-organism. In other words, the total amount of fermentable carbohydrate is the sum of the amount of fermentable carbohydrate that is present in the fermentation medium that is provided in step a) and the amount of fermentable carbohydrate that is added during incubation (e.g. in a fed-batch operation).

The total amount of sodium cation provided in the fermentation medium as referred to herein equals the sum of the amount sodium cation that is present in the fermentation medium that is provided in step a) and the amount of sodium cation that is added during incubation. The same holds for the total amount of potassium cation that is provided in the fermentation medium and the total amount of calcium cation that is provided in the fermentation medium.

In the present process lactate is introduced in the fermentation medium in the form of sodium lactate and/or potassium lactate and/or calcium lactate. This may be achieved by introducing these salts as such into the fermentation medium. Alternatively, these lactates may be introduced by introducing lactic acid into the fermentation medium, followed by addition of a neutralising amount of an alkaline sodium salt (e.g. sodium hydroxide) and/or an alkaline potassium salt (e.g. potassium hydroxide) and/or an alkaline calcium salt (e.g. calcium hydroxide).

Monosaccharide in the fermentation medium can be provided in the form of pure glucose and/or pure fructose. Monosaccharide may also be provided in less pure forms, such as, vegetable juice, vegetable juice concentrate, fruit juice, fruit juice concentrate and starch hydrolysate (e.g. glucose syrup).

It is noted that the present invention also encompasses processes in which glucose and/or fructose containing disaccharides or oligosaccharides are provided in the fermentation medium together with one or more enzymes that are capable of hydrolysing these disaccharides or oligosaccharides before and/or during incubation to thereby release glucose and/or fructose. Examples of such saccharide/enzyme combinations are:

- sucrose + invertase

- maltose syrup + amyloglucosidase

In order to achieve a high yield of propionate, it is advantageous to continue incubation step c) until virtually all fermentable carbohydrate has been digested. Accordingly, in a preferred embodiment, incubation is continued until the combined concentration of monosaccharide and lactate in the fermentation medium has dropped below 17 mmol/L, more preferably below 9 mmol/L and most preferably below 3 mmol/L.

Incubation step c) is preferably continued until the propionate concentration in the fermentation medium has increased to at least 100 mmol/L, more preferably to 150-500 mmol/L and most preferably to 250-350 mmol/L.

In the present process, the total amount of monosaccharide that is provided in the fermentation medium is preferably in the range of 50 to 800 mmol/L, more preferably in the range of 80 to 600 mmol/L, most preferably in the range of 110 to 500 mmol/L, said amounts being calculated on the basis of the volume of clarified liquid fermentate.

Preferably, the monosaccharide that is provided in the fermentation medium is glucose. Accordingly, in a preferred embodiment, the total amount of glucose that is provided in the fermentation medium is preferably in the range of 50 to 800 mmol/L, more preferably in the range of 80 to 600 mmol/L, most preferably in the range of 110 to 500 mmol/L, said amounts being calculated on the basis of the volume of clarified liquid fermentate.

The total amount of lactate provided in the fermentation medium is preferably in the range of 40 to 900 mmol/L, more preferably in the range of 70 to 700 mmol/L, most preferably in the range of 90 to 600 mmol/L, said amounts being calculated on the basis of the volume of clarified liquid fermentate.

The molar ratio between the total amount of monosaccharide provided in the fermentation medium and the total amount of lactate provided in the fermentation medium is preferably in the range of 1:1.9 to 5:1, more preferably in the range of 1:1.8 to 4:1, even more preferably in the range of 1:1.7 to 2:1, most preferably in the range of 1:1.65 to 1:1.

Examples of propionic acid producing micro-organism that may be employed in the present process include *Acidopropionibacterium acidipropionici*, *Propionibacterium freudenreichii*, *Propionibacterium shermanii*, *Acidopropionibacterium thoenii*, *Acidopropionibacterium jensenii* and combinations thereof. Most preferably, the propionic acid micro-organism used is *Acidopropionibacterium acidipropionici.*

Incubation step c) is preferably carried out under anaerobic conditions.

The fermentation medium is preferably incubated at a temperature in the range of 20 to 45 °C, more preferably of 25 to 42 °C and most preferably of 30 to 40 °C.

The duration of the incubation step is preferably in the range of 20 to 120 hours, more preferably in the range of 24 to 72 hours.

The alkalising agent that is employed in the present process to stabilise the pH of the fermentation medium during incubation is preferably selected from alkaline alkalimetal salt, ammonia and combinations thereof. More preferably, the alkaline alkalimetal salt is selected from sodium hydroxide, potassium hydroxide and combinations thereof. Most preferably, the alkaline alkalimetal salt is sodium hydroxide.

According to a particularly preferred embodiment of the present process, the alkalising agent is added to the fermentation medium to maintain pH within the range of 5.5 to 7.2, more preferably within the range of 5.8 to 7.0.

The total amount of sodium cation provided in the fermentation medium in the course of steps a), b) and c) equals X mmol/L. Preferably, $X \geq 100$ mmol/L, more preferably $200$ mmol/L $\leq X \leq 500$ mmol/L, most preferably $300$ mmol/L $\leq X \leq 480$ mmol/L. In accordance with a preferred embodiment $X/(Y+2Z) \geq 3$, more preferably $5 \leq X/(Y+2Z) \leq 1000$.

The total amount of potassium cation provided in the fermentation medium in the course of steps a), b) and c) equals Y mmol/L Preferably, $Y \geq 100$ mmol/L, more preferably $200$ mmol/L $\leq Y \leq 500$ mmol/L, most preferably $300$ mmol/L $\leq Y \leq 480$ mmol/L. In accordance with a preferred embodiment $Y/(X+2Z) \geq 3$, more preferably $5 \leq Y/(X+2Z) \leq 1000$.

Preferably, not more than a small amount of calcium cation is provided in the fermentation medium in the course of steps a), b) and c). Preferably $Z \leq 100$ mmol/L, more preferably $0$ mmol/L $\leq Z \leq 60$ mmol/L, most preferably $0$ mmol/L $\leq Z \leq 30$ mmol/L. In accordance with a preferred embodiment $0 \leq Z/(X+Y) \leq 0.5$, more preferably $0 \leq Z/(X+Y) \leq 0.2$.

The fermentable carbohydrates monosaccharide and lactate may both be present in the fermentation medium at the start of incubation step c). However, it is also feasible to start incubation with a fermentation medium that contains only one of these two carbohydrate substrates and to add the other carbohydrate substrate during incubation. It is further feasible to start with only one of the two carbohydrate substrates and to add both carbohydrate substrates during incubation. Addition of the two carbohydrate substrates during incubation can occur simultaneously, sequentially or alternatingly.

Preferably, the fermentation medium that is inoculated with the propionic acid producing micro-organism contains at least 50 mmol/L of monosaccharide, more preferably 80-600 mmol/L of monosaccharide and most preferably 110-500 mmol/L of monosaccharide.

According to a preferred embodiment, the fermentation medium that is inoculated with the propionic acid producing micro-organism contains at least 50 mmol/L of glucose, more preferably 80-600 mmol/L of glucose and most preferably 110-500 mmol/L of glucose.

In another preferred embodiment of the present invention, lactate is present in the fermentation medium that is inoculated with the propionic acid producing micro-organism and/or lactate is added to the fermentation medium at least 10 hours before removal of the biomass. Lactate may suitably be added during incubation (fed-batch). It is noted that lactate may also be added by separately adding (i) lactic acid and (ii) an alkaline water-soluble salt of sodium, potassium and/or calcium.

According to a preferred embodiment of the present process, lactate is introduced in the fermentation medium in the form of sodium lactate and/or potassium lactate. Most preferably, the lactate is introduced in the fermentation medium in the form of sodium lactate.

The fermentation medium that is provided in step a) of the present process preferably has a water content of at least 85 wt.%, more preferably a water content of at least 90 wt.% and most preferably a water content of 90 to 97 wt.%.

In step d) of the present process biomass may be removed by solid-liquid separation techniques known in the art, such as centrifugation, filtration, decanting and hydrocyclones. Most preferably, the biomass is removed by means of centrifugation.

The clarified liquid fermentate that is obtained in step d) preferably has a dry matter content of 2-8 wt.%, more preferably of 2.5-6.0 wt.%.

The clarified liquid fermentate preferably has a turbidity of less than 100 Nephelometric Turbidity Units (NTU), more preferably of less than 50 NTU. Turbidity can suitably be determined using a turbidity meter such as the WTW™ TURB™ Series 430 portable turbidity meter supplied by Fisher Scientific.

The present process offers the advantage that a clarified liquid fermentate of low turbidity can be obtained by centrifugation. Thus, the clarified fermentate does not require an additional filtration step to reduce turbidity to acceptable levels. Accordingly, in a preferred embodiment, the present process does not include a filtration step after the removal of biomass by means of centrifugation.

In an alternative advantageous embodiment, the clarified liquid fermentate that is obtained by centrifugation is subjected to a filtration step to obtain a clarified liquid fermentate having an extremely low turbidity, e.g. a turbidity of less than 50 Nephelometric Turbidity Units (NTU), more preferably of less than 20 NTU.

Following removal of biomass, the present process preferably comprises the additional step of preparing a concentrated fermentate by concentrating the clarified liquid fermentate to a dry matter content of 40-65 wt.%, more preferably a dry matter content of 45-63 wt.%. Concentration of the fermentate may be achieved by well-known techniques such as evaporation, membrane filtration, freeze concentration or a combination of these techniques. Most preferably, concentration is achieved by evaporation.

According to a further preferred embodiment, lactic acid is added to the concentrated fermentate to lower the pH. Preferably, lactic acid is added to the concentrated fermentate in a concentration of 20 to 100 g/L, more preferably in a concentration of 40 to 85 g/L.

The concentrated fermentate to which lactic acid has been added to lower pH, preferably has a pH in the range of 4.0 to 6.0, more preferably in the range of 4.5 to 5.8 when diluted 1:9 (v/v) with demi-water

The present process may be carried out in a batch-wise, a semibatch-wise or a continuous, fashion. Preferably, the process is carried out in a batch-wise or semibatch-wise fashion.

In accordance with a particularly preferred embodiment, the present process produces a liquid fermentate as specified herein below.

Another aspect of the invention relates to a liquid fermentate having a dry matter content of at least 15 g/L and comprising:

- a mmol of propionate per gram of dry matter;
- b mmol of acetate per gram of dry matter;
- c. mmol of succinate per gram of dry matter;
- x mmol of $Na^+$ per gram of dry matter;
- y mmol of $K^+$ per gram of dry matter;
- z mmol of $Ca^{2+}$ per gram of dry matter;

said fermentate comprising DNA originating from a propionic acid producing micro-organism; wherein:

- $$6 \leq a \leq 9;$$
- $$1 \leq b \leq 5;$$
- $$0.1 \leq c \leq 1.0;$$
- $$8 \leq x \leq 18;$$
- $$8 \leq y \leq 18;$$
- $$4 \leq z \leq 9;$$
- $$c/a \leq 0.1;$$

- $$0.5 \leq (a+b+2c)/(x+y+2z) \leq 1.2.$$

The liquid fermentate of the present invention preferably has a dry matter content of 20-65 g/L, more preferably of 25-60 g/L.

The liquid fermentate preferably has a turbidity of less than 300 Nephelometric Turbidity Units (NTU), more preferably of less than 200 NTU.

Propionate is preferably contained in the liquid fermentate in a concentration of 6.2-8.5 mmol per gram of dry matter, more preferably in a concentration of 6.5-8.2 mmol per gram of dry matter.

Acetate is preferably contained in the liquid fermentate in a concentration of 1.5-4.0 mmol per gram of dry matter, more preferably in a concentration of 2.0-3.5 mmol per gram of dry matter.

Succinate is preferably contained in the liquid fermentate in a concentration of 0.2-0.7 mmol per gram of dry matter, more preferably in a concentration of 0.3-0.6 mmol per gram of dry matter.

According to a particularly preferred embodiment, succinate and propionate are present in the fermentate in a molar ratio of less than 1:12, more preferably in a molar ratio of less than 1:15.

Besides propionate, the liquid fermentate of the present invention preferably contains a substantial amount of acetate, since acetate can enhance the anti-microbial action of the propionate. Preferably, acetate and propionate are present in the liquid fermentate in a molar ratio of at least 1:3.3, more preferably in a molar ratio of 1:3.0 to 1:2.0.

Sodium cation and/or potassium cation are preferably contained in the liquid fermentate in a combined concentration of 9-15 mmol per gram of dry matter, more preferably in a concentration of 10-14 mmol per gram of dry matter.

Sodium cation is preferably contained in the liquid fermentate in a concentration of 9-15 mmol per gram of dry matter, more preferably in a concentration of 10-14 mmol per gram of dry matter. In accordance with a preferred embodiment $x/(y+2z) \geq 3$, more preferably $5 \leq x/(y+2z) \leq 1000$.

Potassium cation is preferably contained in the liquid fermentate in a concentration of 9-15 mmol per gram of dry matter, more preferably in a concentration of 10-14 mmol per gram of dry matter. In accordance with a preferred embodiment $y/(2x+2z) \geq 3$, more preferably $5 \leq y/(x+2z) \leq 1000$.

The liquid fermentate of the present invention preferably contains no more than a marginal amount of $Ca^{2+}$. Preferably, calcium cation is contained in the liquid fermentate in a concentration of 0-2 mmol per gram of dry matter, more preferably in a concentration of 0-0.5 mmol per gram of dry matter. In accordance with a preferred embodiment $0 \leq z/(x+y) \leq 0.1$, more preferably $0 \leq z/(x+y) \leq 0.02$.

The DNA originating from a propionic acid producing micro-organism that is contained in the liquid fermentate preferably originates from one or more propionic acid producing micro-organisms selected from *Acidopropionibacterium acidipropionici, Acidopropionibacterium freudenreichii, Acidopropionibacterium shermanii, Acidopropionibacterium thoenii* and *Acidopropionibacterium jensenii.* More preferably, the DNA originates from propionic acid producing micro-organism selected from ... and combinations thereof. Most preferably, the DNA originates from *Acidopropionibacterium acidipropionici.*

According to a particularly preferred embodiment, the liquid fermentate is obtainable, more preferably obtained by the fermentation process that has been specified herein above.

A further aspect of the invention relates to propionate concentrate having a dry matter content of 40-65 wt.% and comprising:

- 1.5-3.5 mol/L of propionate;
- 0.4-2 mol/L of acetate;
- 80-240 mmol/L of succinate;
- 0.55-2.2 mol/L of lactate;
- x' mol/L of $Na^+$;
- y' mol/L of $K^+$;
- z' mol/L of $Ca^{2+}$;

said concentrate having a pH in the range of 4.0-6.0 when diluted 1:9 (v/v) with demi-water, and comprising DNA originating from a propionic acid producing micro-organism;
wherein succinate and propionate are present in the concentrate in a molar ratio of less than 1:10;
wherein $2.7 \leq x'+y'+2z' \leq 6$.

The propionate concentrate of the present invention may suitably be produced by the embodiment of the process of the present invention in which, following removal of biomass, the fermentate is concentrated and subsequently acidified with lactic acid.

The propionate concentrate of the present invention preferably has a dry matter content of 45-63 wt.%, more preferably of 48-62 wt.%.

Propionate is preferably contained in the propionate concentrate in a concentration of 1.7-3.3 mol/L, more preferably in a concentration of 2.0-3.0 mol/L.

Acetate is preferably contained in the propionate concentrate in a concentration of 0.5-1.7 mol/L, more preferably in a concentration of 0.55-1.5-1.7 mol/L.

Succinate is preferably contained in the propionate concentrate in a concentration of 100-220 mmol/L, more preferably in a concentration of 120-200 mmol/L.

According to a particularly preferred embodiment, succinate and propionate are present in the fermentate in a molar ratio of less than 1:12, more preferably in a molar ratio of less than 1:15.

Besides propionate, the propionate concentrate of the present invention preferably contains a substantial amount of acetate, since acetate can enhance the anti-microbial action of the propionate. Preferably, acetate and propionate are present in the propionate concentrate in a molar ratio of at least 1:2.9, more preferably in a molar ratio of 1:2.8 to 1:2.0.

Sodium cation and/or potassium cation are preferably contained in the propionate concentrate in a combined concentration of 3.0-5.5 mol/L, more preferably in a combined concentration of 3.5-5.2 mol/L.

Sodium cation is preferably contained in the propionate concentrate in a concentration of 3.0-5.5 mol/L, more preferably in a combined concentration of 3.5-5.2 mol/L. In accordance with a preferred embodiment $x'/(y'+2z') \geq 3$, more preferably $x'/(y'+2z') \geq 5$.

Potassium cation is preferably contained in the propionate concentrate in a concentration of 3.0-5.5 mol/L, more preferably in a combined concentration of 3.5-5.2 mol/L. In accordance with a preferred embodiment $y'/(x'+2z') \geq 3$, more preferably $y'/(x'+2z') \geq 5$.

The propionate concentrate of the present invention preferably contains no more than a marginal amount of $Ca^{2+}$. Preferably, calcium cation is contained in the propionate concentrate in a concentration of 0-0.5 mol/L, more preferably in a concentration of 0-0,1 mol/L. In accordance with a preferred embodiment $0 \leq z'/(x'+y') \leq 0.1$, more preferably $0 \leq z'/(x'+y') \leq 0.02$.

The DNA originating from a propionic acid producing micro-organism that is contained in the propionate concentrate preferably originates from one or more propionic acid producing micro-organisms selected from *Acidopropionibacterium acidipropionici, Acidopropionibacterium freudenreichii, Acidopropionibacterium shermanii, Acidopropionibacterium thoenii* and *Acidopropionibacterium jensenii*. More preferably, the DNA originates from propionic acid producing micro-organism selected from ... and combinations thereof. Most preferably, the DNA originates from *Acidopropionibacterium acidipropionici*.

The propionate concentrate preferably has a pH in the range of 4.5-5.8, most preferably in the range of 4.8 to 5.7 when diluted 1:9 (v/v) with demi-water.

The propionate concentrate according to the present invention preferably is a clear liquid, more particularly a liquid having a turbidity of less than 700 Nephelometric Turbidity Units (NTU), more preferably of less than 500 NTU, most preferably less than 300 NTU.

Yet another aspect of the invention relates to a method of preparing a product selected from a food product, a nutritional product and a beverage, said method comprising application of the propionate concentrate of the present invention in a concentration of providing 5-100 mmol propionate per kg of the final product, more preferably providing 10-60 mmol propionate per kg.

According to a particularly preferred embodiment, the present method is used to prepare a bakery product or a processed meat product.

The invention is further illustrated by the following non-limiting examples.

## EXAMPLES

### Example 1

Liquid propionate-containing fermentates were prepared starting from a fermentation medium that contained glucose as the only fermentable carbohydrate and a fermentation medium that contained fermentable carbohydrate in the form of a combination of glucose and sodium lactate.

The fermentation media were prepared having the composition that is described in Table 1.

**Table 1**

|  | g/kg | |
|---|---|---|
|  | Medium 1 | Medium A |
| Glucose | 22.5 | 45 |
| Sodium lactate | 22.5 | 0 |
| Amino acids | 0.25 | 0.25 |
| Yeast extract | 5 | 5 |
| Vitamins & minerals | 0.03 | 0.03 |
| Inoculum | 75 | 75 |
| Water | Remainder | Remainder |

Both fermentation media were inoculated with A. *acidipropionici* and incubated at 35°C for 46 hours under anaerobic condition and continuous agitation. During incubation, pH was maintained at about 6.5 until glucose concentration reached 6 g/L by addition of $NH_3$ and NaOH solutions. Next, fermentation was allowed to continue to consume most of the remaining glucose and to reduce pH.

Biomass was removed from the two fermentates by means of centrifugation, using a disc-stack centrifuge.

The clarified fermentates so obtained were analysed. The results are shown in Tables 2 and 3.

**Table 2**

|  | g/L | |
|---|---|---|
|  | Fermentate 1 | Fermentate A |
| Dry matter | 39 | 39 |
| Glucose | < 1 | < 1 |
| Lactate | < 1 | < 1 |
| Propionate | 21.0 | 17.5 |
| Succinate | 2.0 | 3.2 |
| Acetate | 6.5 | 4.8 |
| Pyruvate | 0.5 | 0.2 |

**Table 3**

|  | Fermentate 1 | Fermentate A |
|---|---|---|
| Propionate (mol/kg) | 0.275 | 0.224 |
| Succinate (mol/kg) | 0.017 | 0.028 |
| Acetate (mol/kg) | 0.105 | 0.077 |
| Succinate:propionate (mol/mol) | 0.062 | 0.122 |
| Acete:propionate (mol/mol) | 0.381 | 0.347 |

Clarified fermentate 1 was concentrated by means of evaporation, followed by sterilisation. Next, the clarified fermentate was acidified by adding natural L-lactic acid (PURAC® FCC 88). The acidified product so obtained had a pH of 5.3 when diluted to 10% (w/v) with demineralised water. The acidified product was again analysed. The results are shown in Table 4.

**Table 4**

| Dry matter (wt.%) | 56 |
|---|---|

(continued)

| | |
|---|---|
| Propionate (wt.%) | 19 |
| Succinate (wt.%) | 1 |
| Acetate (wt.%) | 6 |
| Lactate (wt.%) | 12 |
| Sodium (wt.%) | 9 |
| pH at 10% dilution | 5.3 |
| Colour | Brown |
| Turbity (NTU) | 100 |

**Example 2**

A concentrated liquid propionate fermentate was produced in the same way as the concentrated liquid propionate fermentate of Example 1, except that sodium lactate was replaced by potassium lactate.

The clarified fermentate was analysed. The results are shown in Tables 5 and 6.

**Table 5**

| | g/L |
|---|---|
| Glucose | < 1 |
| Lactate | < 1 |
| Propionate | 20.9 |
| Succinate | 2.0 |
| Acetate | 7.3 |
| Pyruvate | 0.2 |

**Table 6**

| | |
|---|---|
| Propionate (mol/kg) | 0.272 |
| Succinate (mol/kg) | 0.016 |
| Acetate (mol/kg) | 0.121 |
| Succinate:propionate (mol/mol) | 0.057 |
| Acete:propionate (mol/mol) | 0.442 |

**Claims**

1. A process of producing a liquid propionate-containing fermentate, said process comprising:

   a) providing a fermentation medium comprising fermentable carbohydrates selected from monosaccharide, lactate and combinations thereof, said monosaccharide being selected from glucose, fructose and combinations thereof, said lactate being selected from sodium lactate, potassium lactate, calcium lactate and combinations thereof;
   b) inoculating the fermentation medium with a propionic acid producing micro-organism;
   c) incubating the inoculated aqueous liquid for at least 20 hours to produce a fermentate while adding an alkalising agent to the fermentation medium to maintain pH within the range of 5.0 to 7.5; and
   d) removing biomass from the fermentate to produce a clarified liquid fermentate having a dry matter content of at least 1 wt.%;

wherein the total amount of monosaccharide provided in the fermentation medium in the course of steps a), b) and c) exceeds 30 mmol/L and the total amount of lactate provided in the fermentation medium in the course of steps a), b) and c) exceeds 50 mmol/L, said total amounts being calculated on the basis of the volume of the clarified liquid fermentate;

wherein the molar ratio between the total amount of monosaccharide provided in the fermentation medium and the total amount of lactate provided in the fermentation medium is in the range of 1:2.0 to 5.6:1;

wherein the total amount of sodium cation provided in the fermentation medium in the course of steps a), b) and c) equals X mmol/L, the total amount of potassium cation provided in the fermentation medium in the course of steps a), b) and c) equals Y mmol/L and the total amount of calcium cation provided in the fermentation medium in the course of steps a), b) and c) equals Z mmol/L, said total amounts being calculated on the basis of the volume of the clarified liquid fermentate; and

wherein $X + Y + 2Z \geq 100$ mmol/L.

2. Process according to claim 1, wherein incubation step c) is continued until the combined concentration of monosaccharide and lactate in the fermentation medium has dropped below 17 mmol/L.

3. Process according to any one of the preceding claims, wherein the propionic acid producing micro-organism is selected from *Acidopropionibacterium acidipropionici, Propionibacterium freudenreichii, Propionibacterium shermanii, Acidopropionibacterium thoenii, Acidopropionibacterium jensenii* and combinations thereof.

4. Process according to any one of the preceding claims, wherein the alkalising agent is selected from alkaline alkalimetal salt, earth alkaline metal salt, ammonium salt and combinations thereof.

5. Process according to claim 4, wherein the alkaline alkalimetal salt is selected from sodium hydroxide, potassium hydroxide and combinations thereof.

6. Process according to any one of the preceding claims, wherein the fermentation medium that is inoculated with the propionic acid producing micro-organism contains at least 50 mmol/L of monosaccharide

7. Process according to claim 6, wherein lactate is present in the fermentation medium that is inoculated with the propionic acid producing micro-organism and/or wherein lactate is added to the fermentation medium at least 10 hours before removal of the biomass.

8. Processs according to any one of the preceding claims, wherein lactate is introduced in the fermentation medium in the form of sodium lactate and/or potassium lactate.

9. Process according to any one of the preceding claims, wherein the process comprises the additional step of preparing a concentrated fermentate by concentrating the clarified liquid fermentate to a dry matter content of 40-65 wt.%.

10. Process according to any one of the preceding claims, wherein lactic acid is added to the concentrated fermentate in a concentration of 50 to 200 g/L.

11. A liquid fermentate having a dry matter content of at least 15 g/L and comprising:

    • a mmol of propionate per gram of dry matter;
    • b mmol of acetate per gram of dry matter;
    • c. mmol of succinate per gram of dry matter;
    • x mmol of $Na^+$ per gram of dry matter;
    • y mmol of $K^+$ per gram of dry matter;
    • z mmol of $Ca^{2+}$ per gram of dry matter;
said fermentate comprising DNA originating from a propionic acid producing micro-organism;
wherein:

    •

$$6 \leq a \leq 9;$$

- 
$$1 \leq b \leq 5;$$

- 
$$0.1 \leq c \leq 1.0;$$

- 
$$8 \leq x \leq 18;$$

- 
$$8 \leq y \leq 18;$$

- 
$$4 \leq z \leq 9;$$

- 
$$c/a \leq 0.1;$$

- 
$$0.5 \leq (a+b+2c)/(x+y+2z) \leq 1.2.$$

12. Liquid fermentate according to claim 11, wherein $b/a \geq 1{:}2.9$.

13. A propionate concentrate having a dry matter content of 40-65 wt.% and comprising:

- 1.5-3.5 mol/L of propionate;
- 0.4-2 mol/L of acetate;
- 80-240 mmol/L of succinate;
- 0.55-2.2 mol/L of lactate;
- $x'$ mol/L of $Na^+$;
- $y'$ mol/L of $K^+$;
- $z'$ mol/L of $Ca^{2+}$;

said concentrate having a pH in the range of 4.0-6.0 when diluted 1:9 (v/v) with demi-water, and comprising DNA originating from a propionic acid producing micro-organism; wherein succinate and propionate are present in the concentrate in a molar ratio of less than 1:10;

wherein $2.7 \leq x'+y'+2z' \leq 6$.

14. Propionate concentrate according to claim 13, wherein the concentrate contains at least 2.7 mol/L of $Na^+$ and/or $K^+$.

15. A method of preparing a product selected from a food product, a nutritional product and a beverage, said method comprising application of the propionate concentrate according to claim 13 or 14 in a concentration providing 5-100 mmol propionate per kg of the final product.

Europäisches
Patentamt

European
Patent Office

Office européen
des brevets

## PARTIAL EUROPEAN SEARCH REPORT

under Rule 62a and/or 63 of the European Patent Convention.
This report shall be considered, for the purposes of
subsequent proceedings, as the European search report

**Application Number**

EP 23 16 8563

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Martinez-Campos Roberto ET AL: "Production of propionate by fed-batch fermentation of Propionibacterium acidipropionici using mixed feed of lactate and glucose", Biotechnology letters, March 2002 (2002-03), pages 427-431, XP093094194, Dordrecht DOI: 10.1023/A:1014562504882 Retrieved from the Internet: URL:https://link.springer.com/content/pdf/10.1023/A:1014562504882.pdf?pdf=inline%20link [retrieved on 2023-10-23] * Materials and Methods; figures 1-4 * * Discussion * | 1-10, 13-15 | INV. A23L2/44 A23L3/3508 C12P7/52 |
| X | EP 3 140 416 B1 (ACIES BIO D O O [SI]; ARIMA D O O [SI]) 1 August 2018 (2018-08-01) * examples 2-4 * -/-- | 1-10, 13-15 | TECHNICAL FIELDS SEARCHED (IPC) C12P A23L |

### INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

**see sheet C**

| Place of search | Date of completion of the search | Examiner |
|---|---|---|
| The Hague | 24 October 2023 | Granet, Nicolas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

Europäisches
Patentamt

European
Patent Office

Office européen
des brevets

**PARTIAL EUROPEAN SEARCH REPORT**

Application Number

EP 23 16 8563

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| A | US 5 096 718 A (AYRES JAMES W [US] ET AL) 17 March 1992 (1992-03-17) * examples 2-4 * ----- | 1-10, 13-15 | |
| X | LEE INN HEE ET AL: "Diauxic Growth of Propionibacterium shermanii", APPLIED MICROBIOLOGY, vol. 28, no. 5, November 1974 (1974-11), pages 831-835, XP093094212, US ISSN: 0003-6919, DOI: 10.1128/am.28.5.831-835.1974 Retrieved from the Internet: URL:https://journals.asm.org/doi/pdf/10.1128/am.28.5.831-835.1974> * Materials and Methods; figures 1-6 * * Discussion * ----- | 1-10, 13-15 | TECHNICAL FIELDS SEARCHED (IPC) |

EPO FORM 1503 03.82 (P04C10)

1

Europäisches
Patentamt

European
Patent Office

Office européen
des brevets

**INCOMPLETE SEARCH
SHEET C**

Claim(s) completely searchable:
       1-10, 13-15

Claim(s) not searched:
       11, 12

Reason for the limitation of the search:

Claims 11 and13 (product claims) have been drafted as separate
independent claims.
Under Article 84 in combination with Rule 43(2) EPC, an application may
contain more than one independent claim in a particular category only if
the subject-matter claimed falls within one or more of the exceptional
situations set out in paragraph (a), (b) or (c) of Rule 43(2) EPC. This
is not the case in the present application, however, for the following
reasons:
i) the criteria a) is not met: it is not clear what is the link between
claims 11 and 13. Indeed, the concentrate disclosed in claim 13 is not
the concentrate obtained from the liquid fermentate of claim 11 but
rather seems to relate to another distinctive product obtained from
another liquid fermentate than the one disclosed in claim 11.
Indeed, when using the dry matter content of claim 11 and trying to
extrapolate based on the dry matter of claim 13, the obtained quantity of
propionate falls outside the range of current claim 13. The same applies
to other ranges.
ii) since they are product claims, they do not relate to different uses.
iii) the criteria c) is not met: they do not seem to relate to
alternative solutions and it would not seem inappropriate to cover these
alternatives by a single claim, or at least by specifying that the
concentrate is obtained from the liquid fermentate of claim 11 and
adapting the concentration ranges accordingly.

In his letter of 13-10-2023, the applicant argued that claims 11 and 13
were falling under the exception of Rule 43(2)(a) EPC, since both claims
11 and 13 were interrelated products and more specifically intermediate
and final chemical products. There is however no evidence provided by the
applicant demonstrating such a link. In fact as already explained above,
the products of claims 11 and 13 seem to relate to different products.
The search has been restricted to the subject-matter indicated by the
applicant as a subsidiary request in his letter of 13-10-2023 filed in
reply to the invitation pursuant to Rule 62a(1), i.e. claims 1-10 and
13-15.
The applicant's attention is drawn to the fact that the application will
be further prosecuted on the basis of subject-matter for which a search
has been carried out and that the claims should be limited to that
subject-matter at a later stage of the proceedings (Rule 63(3 )EPC).

## ANNEX TO THE EUROPEAN SEARCH REPORT
## ON EUROPEAN PATENT APPLICATION NO.

EP 23 16 8563

This annex lists the patent family members relating to the patent documents cited in the above-mentioned European search report.
The members are as contained in the European Patent Office EDP file on
The European Patent Office is in no way liable for these particulars which are merely given for the purpose of information.

24-10-2023

| Patent document cited in search report | | Publication date | Patent family member(s) | | Publication date |
|---|---|---|---|---|---|
| EP 3140416 | B1 | 01-08-2018 | AU | 2015257590 A1 | 10-11-2016 |
| | | | BR | 112016025970 A2 | 15-08-2017 |
| | | | CA | 2948421 A1 | 12-11-2015 |
| | | | CN | 106536715 A | 22-03-2017 |
| | | | DK | 3140416 T3 | 26-11-2018 |
| | | | EA | 201692263 A1 | 28-04-2017 |
| | | | EP | 2942397 A1 | 11-11-2015 |
| | | | EP | 3140416 A1 | 15-03-2017 |
| | | | ES | 2698529 T3 | 05-02-2019 |
| | | | HU | E040087 T2 | 28-02-2019 |
| | | | JP | 6814131 B2 | 13-01-2021 |
| | | | JP | 2017514526 A | 08-06-2017 |
| | | | KR | 20170002620 A | 06-01-2017 |
| | | | PL | 3140416 T3 | 29-03-2019 |
| | | | PT | 3140416 T | 16-11-2018 |
| | | | SI | 3140416 T1 | 31-12-2018 |
| | | | US | 2017145467 A1 | 25-05-2017 |
| | | | WO | 2015169967 A1 | 12-11-2015 |
| | | | ZA | 201608104 B | 24-04-2019 |
| US 5096718 | A | 17-03-1992 | US | 5096718 A | 17-03-1992 |
| | | | US | 5260061 A | 09-11-1993 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010097362 A
- WO 2016146721 A
- WO 2019108064 A